# EUROPEAN PATENT APPLICATION

(11) **EP 2 311 356 A1**
(43) Date of publication of application: **20.04.2011**
(21) Application number: 09758244.9
(22) Date of filing: 27.05.2009
(51) Int. Cl.: A47K 11/06, A61L 9/01, A61L 9/16, C02F 11/00

(54) **METHOD OF TREATING FECES OR EXCRETIONS AND APPARATUS FOR TREATING FECES OR EXCRETIONS**

(30) Priority: 03.06.2008 JP 2008145866
(71) Applicant: Excelsior Inc., Tokyo 154-0023 (JP)
(72) Inventor: ADACHI, Kanichi, Tokyo 154-0023 (JP); ADACHI, Harue, Tokyo 154-0023 (JP)
(74) Representative: Stolmár Scheele & Partner
(86) International application number: PCT/JP2009/059656
(87) International publication number: WO 2009/147977

(57) **Abstract**

[Problem] A method of treating excreta is provided in which excreta is received into a simplified toilet pack and treated in a treatment container usable for a thermal treatment, and the treated excreta can be hygienically discarded.

[Means of solving the problem] It is a method of treating excreta **characterized in** receiving excreta with a container-shaped toilet pack made of a heat-resistant and water-repellent film material containing a heat insulating member at least in the excreta receiving area; sprinkling excreta treatment agent over the excreta received by said toilet pack; throwing them without mixing into a treatment bag made of a heat-resistant film material together with said toilet pack to have the excreta thermally treated inside said treatment bag; and discarding the contents together with the treatment bag.

## Description

### [Technical field]

The present invention relates to a method of excreta treatment and a device for excreta treatment during a time of natural disaster, etc.

### [Background technology]

Toilets of a family residence may become useless during a long term of power outage or water supply shutdown in case of a natural disaster such as an earthquake, which may result in a large accumulation of untreated excreta. It may cause an extreme degradation of living environment due to foul smell exuded from a large accumulation of untreated excreta.

Several methods have been proposed to cope with the problem which may result during a natural disaster, such as a plastic bag to keep excreta temporarily in a sealed condition, but such a plastic bag is prone to be damaged easily, which results in leaking of the contents and end up in further contamination of the environment. Moreover, excreta insides the bag decompose as the time goes inside the bag and the bag cannot prevent the foul gas leaking from it as the internal gas pressure rises.

Other inventions have been disclosed lately such as a simplified throw-away toilet bowl having of a structure of assembled cardboards (see Patent Document 1), and a simplified toilet having a plastic bag, e.g., a polyethylene bag, provided inside a frame structure made of wood or paper, wherein said bag is used only once to be thrown away (see

### Patent Document 2).

On the other hand, several inventions for actively treating excreta have been disclosed such as a method of introducing excreta into saw dusts, agitate the saw dusts and excreta together to treat the excreta with the help of microorganisms residing in the excreta (see Patent Document 3), and an excreta treating agent consisting of fine particles of calcined lime coated with a coating agent selected from a group consisting of higher fatty acids, their salts or esters, and a method of treating excreta with such a treating agent (see Patent Document 4).

### [Prior art document]

### [Patent document]

- [Patent document 1]: Publication of Unexamined Japanese Patent Application 2006-296925.
- [Patent document 2]: Publication of Unexamined Japanese Patent Application 2006-223725.
- [Patent document 3]: Publication of Unexamined Japanese Patent Application H11-300324.
- [Patent document 4]: Publication of Unexamined Japanese Patent Application 2002-210496.

### [SUMMARY]

### [Problems to be solved]

The problem with a throwaway, i.e., one-time-use only, simplified toilet is that discarding it without further treating still contaminates environment as it continues to generate foul smell and is undesirable from a hygienic standpoint. Therefore, it is desirable to apply the treatment methods such as those disclosed in Patent Documents 3 and 4 before discarding the simplified toilets.

However, the treatment method described in Patent Document 3 has a limited capability, e.g., slowness of treatment, as it relies on actions of microorganisms,

On the other hand, the usage of an excreta treatment agent containing fine particles of calcined lime described in Patent Document 4 intending to thermally treating excreta with hydration reaction heat, the throwaway simplified toilets (Patent Documents 1 and 2) may make holes or cause a fire because of the reaction heat. Moreover, causing the user to use a simplified toilet, which is grossly different from a regular toilet the user is familiarized with may cause mental resistance in using it.

The present invention consequently intends to provide a method of treating excreta and a device for thermally treating excreta that are capable of receiving excreta with a simplified toilet pack, treating the excreta inside a treatment container usable for a thermal treatment, and discarding the excreta hygienically.

### [Means to solve the problem]

The object described above can be achieved by the following means:
(1) It is a method of treating excreta characterized in receiving excreta with a container-shaped toilet pack made of a heat-resistant and water-repellent film material containing a heat insulating member at least in the excreta receiving area; sprinkling excreta treatment agent over the excreta received by said toilet pack; throwing them without mixing into a treatment bag made of a heat-resistant film material together with said toilet pack to have the excreta thermally treated inside said treatment bag; and discarding the contents together with the treatment bag.
(2) The method of treating excreta described in section (1) where said toilet pack is formed by thermally fusing a metal foil laminated with a plastic layer into a container shape, and the heat insulating member is embedded at least in its excreta receiving area.
(3) The method of treating excreta described either in section (1) or section (2) where said toilet pack is placed in an existing toilet bowl to receive the excreta.
(4) The method of treating excreta described in either one of section (1) through section (3), wherein said treatment bag is formed by thermally fusing a metal foil material laminated with a plastic layer into a bag shape, with its opening part formed in such a way as to make it sealable.
(5) The method of treating excreta described in section (4) wherein said treatment bag has a ventilation window part, which ventilation window part is equipped with activated charcoal for absorbing foul odor gas existing in the treatment bag and a water-absorbing material that prevents said activated charcoal from absorbing moisture.
(6) The method of treating excreta described in either one of section (1) through section (5), wherein said excreta treatment agent is at least a powdery agent containing a heat generating agent and calcined lime surface-treated with a hydrophobic coating agent.
(7) A device for treating excreta characterized in comprising a container-shaped toilet pack made of a heat-resistant and water-repellent filmmaterial containing a heat insulating member at least in the excreta receiving area; excreta treatment agent to be sprinkled over the excreta received by said toilet pack; and a treatment bag made of a heat-resistant film material, into which said toilet pack is thrown after the sprinkling of said excreta treatment agent to thermally treat the excreta.
(8) The device for treating excreta described in section (7) where said toilet pack is formed by thermally fusing a metal foil laminated with a plastic layer into a container shape, and the heat insulating member is embedded at least in its excreta receiving area.
(9) The device for treating excreta described in section (7) or section (8) wherein said toilet pack has holding parts that can be held by the toilet seat of an existing clam-shell type toilet.
(10) The device for treating excreta described in either one of section (7) through section (9), wherein said treatment bag is formed by thermally fusing a metal foil material laminated with a plastic layer into a bag shape, with its opening part formed in such a way as to make it sealable.
(11) The device for treating excreta described in section (10) wherein said treatment bag has a ventilation window part, which ventilation window part is equipped with activated charcoal for absorbing foul odor gas existing in the treatment bag and a water-absorbing material that prevents said activated charcoal from absorbing moisture.
(12) The device for treating excreta described in either one of section (7) through section (11), wherein said excreta treatment agent is at least a powdery agent containing a heat generating agent and calcined lime surface-treated with a hydrophobic coating agent.

### [Effects of the invention]

According to the present invention, it is possible to receive excreta by a throwaway toilet pack placed on an existing regular toilet, and conduct a thermal treatment of the excreta in a treatment container placed outdoors etc, using an excreta treatment agent containing a heat generating agent and calcined lime surface-treated with, for example, a hydrophobic coating agent. Since the toilet pack formed in a container shape to receive the excreta is made of a water-repellent metal foil and formed by thermal fusion bonding, it prevents water contents of the excreta received by the toilet pack from leaking outside.

Moreover, since the part of the toilet pack which receives excreta has a heat insulating material built into it, an excreta treatment agent is sprinkled over the excreta received by the toilet pack, and the toilet pack containing all those things is thrown into a treatment bag made of a metal foil, so that there is no danger of causing a hole in the treatment bag or starting a fire due to the reaction heat of the excreta treatment agent.

Moreover, since the treatment bag has a ventilation window part, which ventilation window part is equipped with activated charcoal for adsorbing smelly gas existing in the treatment bag and a water-absorbent member that prevents said activated charcoal from absorbing moisture, so that generation the foul smell of the excreta can be prevented.

The thermally treated excreta can be discarded together with the treatment bag so that it is extremely hygienic.

### [Brief description of the drawings]

Fig. 1 is a schematic view showing a film-like material before it is formed into a toilet pack of an excreta treatment device according to an embodiment of the present invention.
Fig. 2A is a schematic view showing a forming procedure of the toilet pack according to the embodiment of the present invention.
Fig. 2B is a schematic view showing a forming procedure of the toilet pack according to the embodiment of the present invention.
Fig. 2C is a schematic view showing a forming procedure of the toilet pack according to the embodiment of the present invention.
Fig. 2D is a schematic view showing a forming procedure of the toilet pack according to the embodiment of the present invention.
Fig. 2E is a schematic view showing a forming procedure of the toilet pack according to the embodiment of the present invention.
Fig. 2F is a schematic view showing a forming procedure of the toilet pack according to the embodiment of the present invention.
Fig. 2G is a schematic view showing a forming procedure of the toilet pack according to the embodiment of the present invention.
Fig. 3 is a schematic view showing a condition where the toilet pack according to the embodiment of the present invention is placed in a toilet bowl.
Fig. 4 is a schematic view showing a condition where the toilet pack according to the embodiment of the present invention is held by a toilet seat.
Fig. 5 is a schematic view showing a condition of sprinkling an excreta treatment agent over the excreta contained in a toilet pack.
Fig. 6 is a schematic view showing a treatment bag according to the embodiment of the present invention.
Fig. 7 is a schematic view showing a condition where the excreta is thrown into the treatment bag according to the embodiment of the present invention together with the toilet pack.

### [Embodiments]

A preferred embodiment of the present invention is described below with reference to the accompanying drawings.

### (Excreta treatment device)

First, let us describe the device for treating excreta used in the method of treating the excreta according to the present invention.

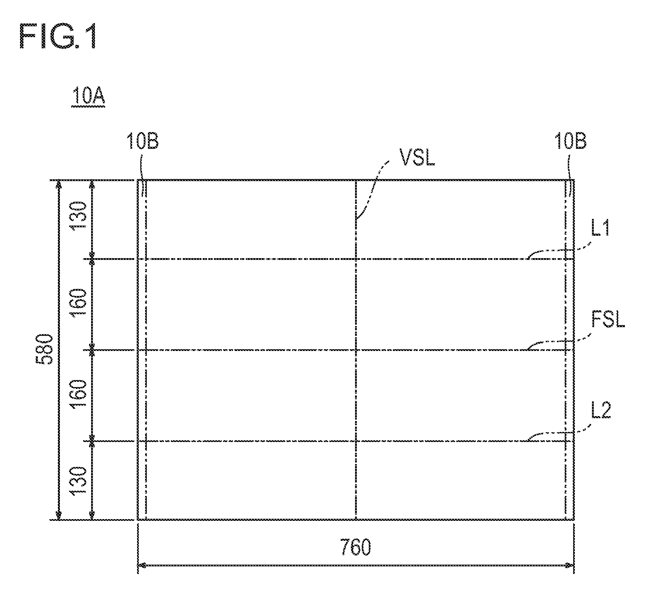

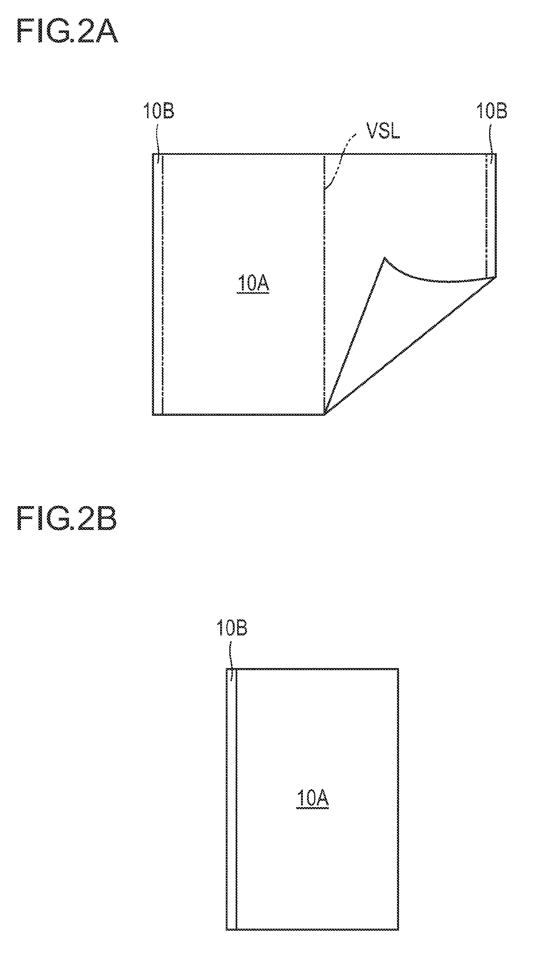

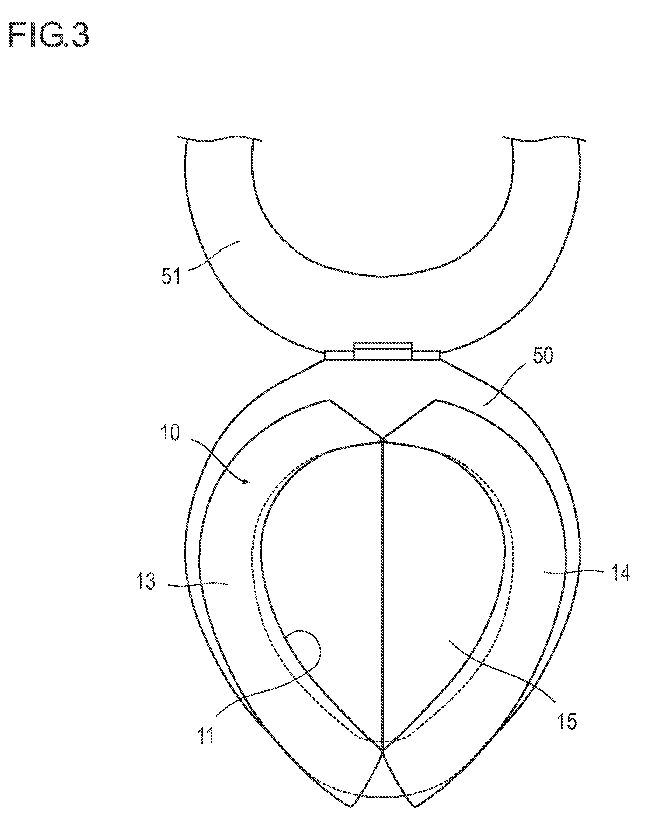

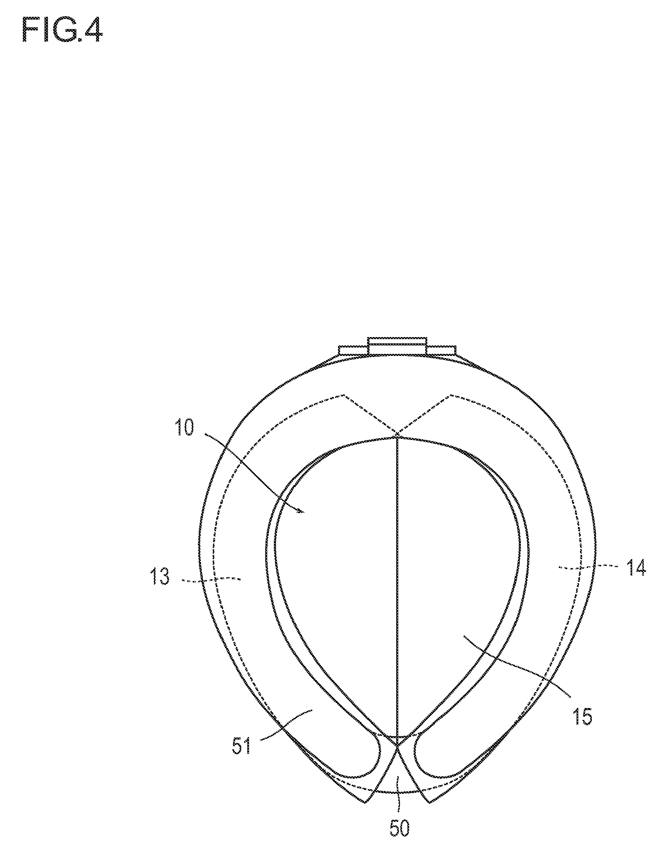

Fig. 1 is a schematic view showing a filmmaterial before it is formed into a toilet pack of an excreta treatment device according to an embodiment of the present invention. Fig. 2A through 2G are schematic views showing a forming procedure of the toilet pack according to the embodiment of the present invention. Fig. 3 is a schematic view showing a condition where the toilet pack according to the embodiment of the present invention is placed in a toilet bowl. Fig. 4 is a schematic view showing a condition where the toilet pack according to the embodiment of the present invention is held by a toilet seat.

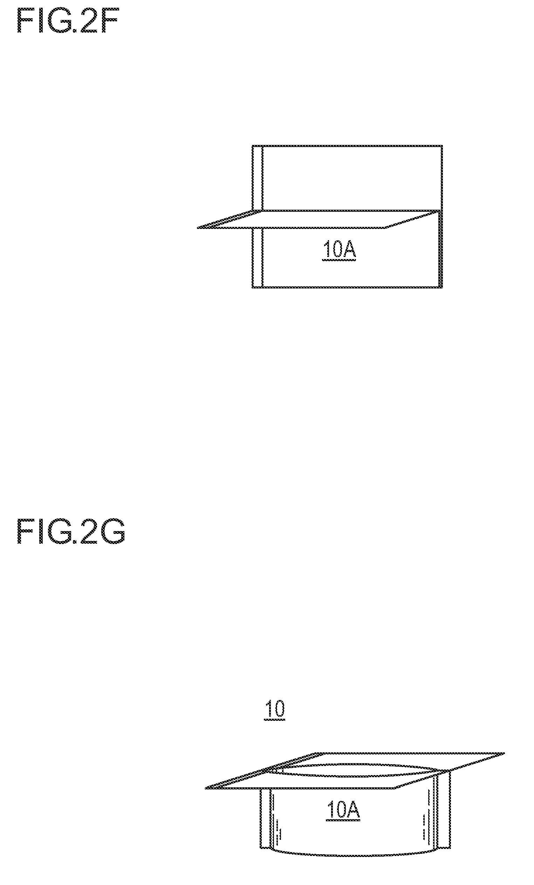

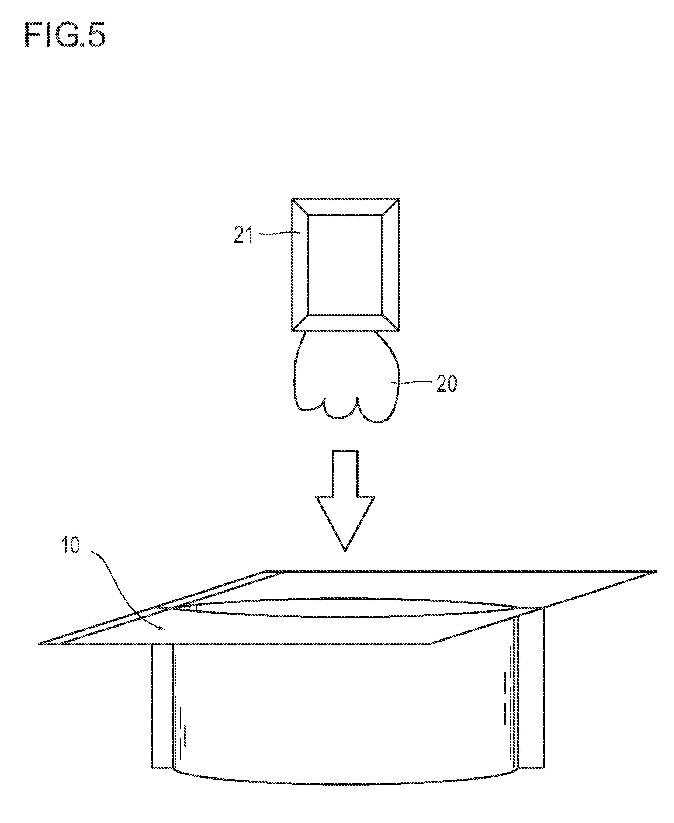

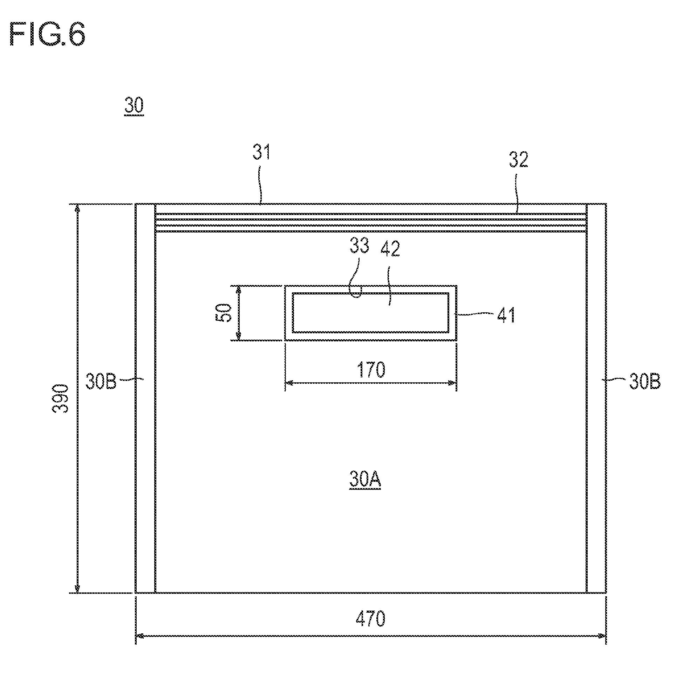

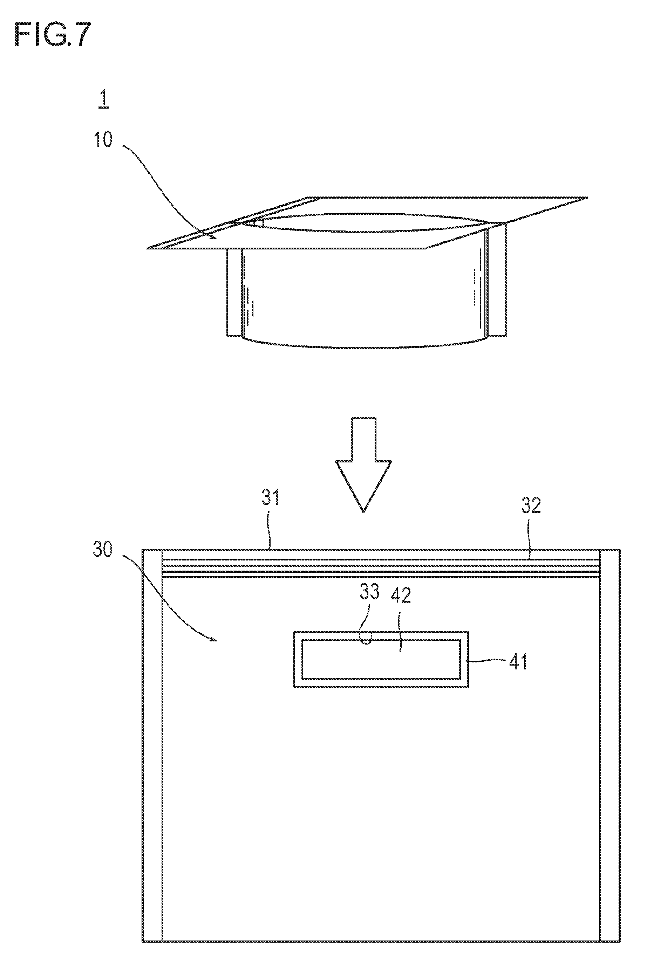

In reference to Fig. 1 and Figs . 2A through 2G, an excreta treatment device 1 according to the present embodiment is equipped with a container-like toilet pack 10, an excreta treatment agent 20 to be sprinkled over the excreta received by the toilet pack 10 (see Fig. 5), and a treatment bag 30 used for thermally treating the excreta which is thrown into the bag together with said toilet pack 10 (see Figs. 6 and 7) after the sprinkling of the excreta treatment agent 20. The toilet pack 10 is a throwaway type simplified container to receive excreta and is formed in a shape of container by folding and thermally fusing a heat-resistant film material laminated with a layer of plastic material on its front and back. This tray pack 10 is stored in a folded condition and is formed in a container shape by expanding its opening edge 11 in such a way that it forms a cavity inside when it is in use (refer to Fig. 2G and Fig. 3).

The film material (base material) 10A of this toilet pack 10 consists of a metal foil, e.g., aluminum foil or copper foil. Since it is made of a metal foil, it provides heat resistance for a temperature range of 80°C to 450°C and hence withstands the reaction heat of the excreta treatment agent 20 to be described later, thus eliminating concerns for making holes or causing fires due to the heat. The reason that the heat resistance at temperatures above 80°C is required is that the system temperature reaches in the neighborhood of 80°C to 95°C because of a hydration reaction of the excreta treatment agent to be described later. The reason that the heat resistance at temperatures below 450°C is required is that the heat is difficult to be transferred in the systembecause the excreta treatment agent 20 to be described later is made of power, and it is deduced that its temperature locally reaches in the neighborhood of 200°C to 400°C because of its hydration reaction.

Moreover, in the present embodiment, although the film material 10A of the toilet pack 10 is water-repellent as it is a metal foil regardless of whether it has plastic layers on the front and back surfaces or not, the front and back surfaces of the metal foil material are coated or laminated with layers of plastics, e.g., polyethylene, polypropylene, nylon, etc. Therefore, it is possible to thermally fuse the film material using a sealer (sealing device) in forming the film material into a container shape, thus providing an excellent sealing capability of the jointed area. Additionally, although the plastic layer is laminated on both the front and back surfaces of the metal foil material in the present embodiment, the invention is not limited to such a configuration; it only requires a plastic layer to be laminated on the jointing surface side of it.

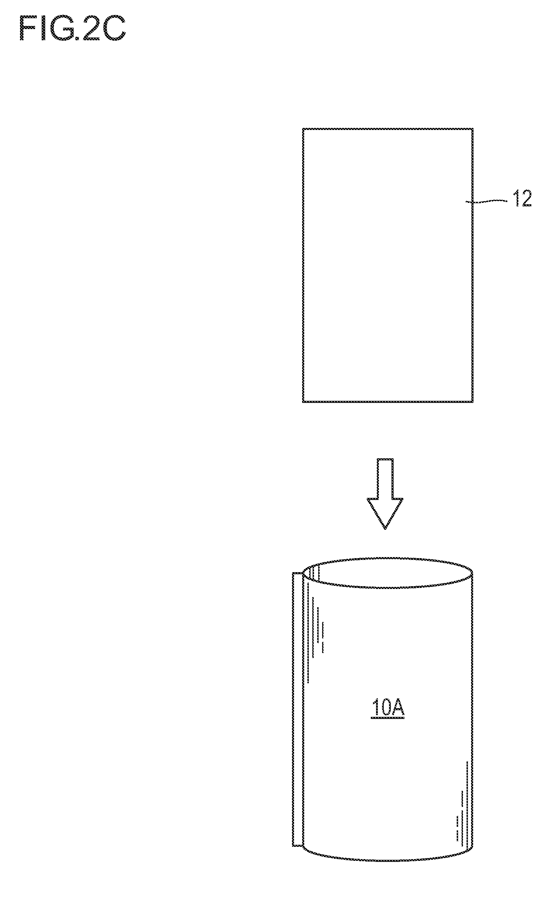

Moreover, a heat insulation material 12 is built into the metal foil material that constitutes the toilet pack 10 (Fig. 2C). The heat insulation material 12 can consist of, for example, heat resistant paper, flame-retardant paper, or glass wool, but it is not restricted to them. Although the heat insulation material 12 is provided for the entire area of the toilet pack 10 in case of the present embodiment, the invention is not limited to it and it can be built at least only in the area that comes in contact with excreta. Since the film material 10A of the toilet pack 10 is made of a metal foil, having the heat insulation material 12 built into the area that comes in contact with excreta can prevent the reaction heat generated by the excreta treatment agent 20 when it is added to the excreta to be transferred to the outer surface of the toilet pack 10. Furthermore, having the heat insulation material 12 built into the area that comes in contact with the excreta adds rigidity to the bottom of the pack so that it adds stability of the pack when it is being formed into a container shape at the time of its use.

Next, let us describe the folding/forming procedure of the toilet pack 10 with reference to Fig. 1, and Figs. 2A - Fig. 2G. This folding structure is only an example of folding/forming the toilet pack 10 to form a container, so that the invention is not limited by its specific structure, size or procedure.

The film material 10A of the toilet pack 10 is an aluminum foil of a 580 mm x 760 mm rectangular shape having a plastic lamination on both the front and back surfaces. The toilet pack 10 assumes lines VSL, FSL, L1 and L2 when it is spread as shown in Fig. 1. Also, a jointing margin 10B is provided on both the left and right edges of the film material 10A for the thermal fusion process as shown in Fig. 1.

First, as shown in Fig. 2A, the film material 10A is folded inward (top side) about the vertical centerline VSL located in the middle of the width direction of the rectangular shape in order to overlay the jointing margins 10B of the left and right edges of the width direction with each other . The jointing margins 10B of both edges are jointed together by thermally fusing them by means of a sealer (not shown) to form a cylindrical shape as shown in Fig. 2B. Next, a heat resistant sheet is inserted as a heat insulation material 12 into the cylindrical aluminum foil material 10A as shown in Fig. 2C.

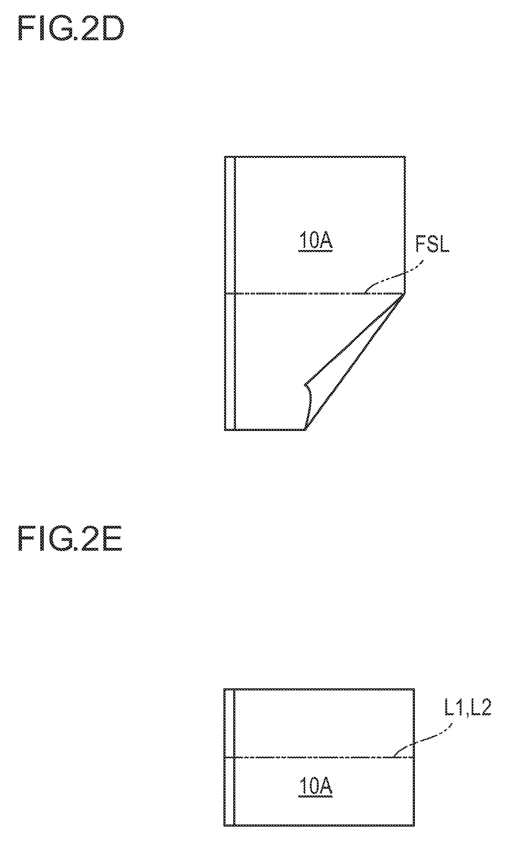

Next, the tubular film material 10A containing the heat resistant sheet 12 is folded inward (top side) about the horizontal center line FSL located in the middle of the vertical direction of the film material 10A in order to overlay the top and bottom edges with each other as shown in Fig 2D.

Next, the top portions of the vertically folded composite material are foldedback using the horizontal lines L1 and L2 as datum lines, to the front and back sides opposite to each other, as shown in Fig. 2E and Fig. 2F. Lastly, the left and right edges of the overlapping bottom half portions are jointed together by thermal fusion using the sealer (not shown) to form a container shape as shown in Fig. 2G.

While the toilet pack 10 is folded in a container shape as is, the toilet pack 10 is formed as a simplified container as the opening edge part F is expanded by holding its folded-back portions 13 and 14 (hereinafter referred to as "holding part") with fingers (see Fig. 3).

As can be seen from Fig. 3 and Fig. 4, the toilet pack 10 according to the present embodiment is equipped with the rectangular wing-like holding parts 13 and 14 on both sides so that it can be held securely underneath a toilet seat 51 of an existing clamshell type toilet (Western style toilet) with the help of the holding parts 13 and 14 after expanding the opening edge 11 sideway by holding the holding parts 13 and 14 with fingers. The toilet pack 10 can also be used by itself without mounting on the existing toilet bowl 50. The procedures of installing the toilet pack 10 on the toilet seat 51 of the existing toilet bowl 50 will be described later.

Fig. 5 is a schematic view showing a condition of sprinkling a excreta treatment agent over the excreta contained in a toilet pack.

In reference to Fig. 5, the excreta treatment agent 20 is stored in a moisture-proof bag 21 in a sealed condition and is sprinkled over the excreta received by the toilet pack 10 by opening the bag 21. The excreta treatment agent 20 can be used by measuring a certain amount in each usage, but it can also be arranged in such a way that an amount appropriate for one or several evacuations is seal-packed in the bag 21 for the sake of convenience in the present embodiment. The excreta treatment agent 20 is a powder agent containing a heat generating agent and calcined lime surface-treated with a hydrophobic coating agent, and it can also contain a water-absorbent member and/or additives as needed. The components of the excreta treatment agent 20 are described below in detail.

### (Calcined lime to be surface-treated)

Although the configuration of calcined lime (CaO) to be surface-treated to be used in the present invention is not particularly specified, it is preferable that it is in a powdered state from the standpoint of its effective dispersion on feces. Its average particle diameter should preferably be in the range of 0.1 µm - 5 mm, or more preferably in the range of 0.5 µm - 2 mm, or most preferably in the range of 1 µm - 1 mm. If the average particle diameter happens to be less than 0.1 µm, production becomes difficult and the production cost may become too expensive. On the other hand, if it is greater than 5 mm, the contact areas between the excreta treatment agent and the excreta reduce, thus resulting in a reduced reaction efficiency, while it may also develop lumpy objects to leave a certain amount of untreated materials.The calcined lime to be surface-treated can consist of a mixture of two different kinds of calcined lime with different average particle diameters as needed. Said average diameter of particles to be used in the present invention is to be determined by a particle size distribution measuring device.

### (Hydrophobic coating agent)

The hydrophobic coating agent to be used in the present invention should preferably be a chemical compound that adds hydrophobic property to calcined lime when calcined lime is surface-treated by using the coating agent and helps the coated calcined lime to become powdery.

An example of said coating agent is, at least, a type of compound selected from a group comprising paraffin, stearylamine, higher fatty acid, salt of higher fatty acid, or ester of higher fatty acid salt, among which it is preferably a type of compound selected from a group comprising higher fatty acid, salt of higher fatty acid, or ester of higher fatty acid salt. As the higher fatty acid to be used here is preferably a monovalent or multivalent saturated fatty acid or unsaturated fatty acid with a carbon number of 8 - 26, or more preferably a monovalent or multivalent saturated fatty acid or unsaturated fatty acid with a carbon number of 8 - 24, or most preferably a monovalent saturated fatty acid with a carbon number of 10 - 20. Specific examples of said higher fatty acids include caprylic acid, pelargonic acid, capric acid, lauric acid, myristic acid, palmitic acid, stearic acid, arachic acid, lignoceric acid, oleic acid, linoleic acid, linolenic acid, and arachidonic acid. Specific examples of said salts of higher fatty acids include alkaline metallic salts including ammonium salt, sodium salt, and potassium salt; or metallic salts of second group including calcium salt and magnesium salt. Specific examples of said esters of higher fatty acids include alkyl esters including methyl ester, ethyl ester, n-propyl ester, isopropyl ester, n-butylester, sec-butyl ester, tert-butyl ester, isobutyl ester, and n-hexyl ester; or cycloalkyl esters including cyclohexyl ester.

The amount of a coating agent to be used is preferably within the range of 0.01-10 mass percentage relative to calcined lime to be surface-treated, or more preferably within the range of 0.1-3 mass percentage, or most preferably within the range of 0.4-1.5 mass percentage. If the amount of a coating agent to be used is less than 0.01 mass percentage, a sufficient delaying effect cannot be achieved which causes a rapid reaction during the excreta treatment, so that lumpy objects develop due to insufficient mixing, and a product of a reaction cannot maintain hydrophobic property and may return to the original form of excreta as water infiltrate in it. On the other hand, if the amount exceeds 10 mass percentage, the active surface area of calcined lime reduces so that the reaction becomes less efficient, and also the process becomes more expensive.

### (Calcined lime surface-treated with hydrophobic coating agent)

The method of producing calcined lime surface-treated with a hydrophobic coating agent is not specifically limited so long as it does not adversely affect the excreta treating agent and the method of treating excreta according to the present invention. For example, the method of mixing calcined lime powder with a coating agent at a temperature higher than the melting point of the coating agent, or the method of spraying a molten coating agent over calcined lime powder kept at a temperature slightly below the melting point of the coating agent while agitating the contents is preferable. It is also preferable to apply the aforementioned coating method after cooling calcined lime to a specified temperature, as calcined lime is normally produced by baking calcium carbonate. The surface-treated calcined lime to be used here can be a mixture of two different kinds of calcined lime surface-treated with different amounts of the coating agent.

Although the physical shape of said surface-treated calcined lime is not specifically limited, it is preferably in a powdery condition. Its average particle diameter should preferably be in the range of 0.1 µm - 5 mm, or more preferably in the range of 0.5 µm - 2 mm, or most preferably in the range of 1 µm - 1 mm,

### (Heat generating agent)

Although the heat generating agent to be used in the present invention is not specifically limited and any known heat generating agent can be used so long as it does not adversely affect the method of treating excreta, it is preferable that it generates a sufficient amount of heat required for melting said coating agent in the excreta treatment system by means of a reaction with water, air, hydrated lime (Ca(OH)2), etc. Specific examples of these heat generating agents include, for example, calcined lime, powdered aluminum, aluminum oxide, magnesium oxide, and anhydrous magnesium chloride. These chemical compounds can be used singularly or as a combination of two or more.

Among these, calcined lime is best suited for this purpose as it can contribute to the treatment of excreta. The amount of calcined lime to be used in this case is preferably 20-200 parts by mass relative to 100 parts by mass of said surface-treated calcined lime. If the amount of calcined lime used in this case is less than 20 parts by mass, the generated amount of heat is too little to cause effective activation of said surface-treated calcine lime so that it may take a longer time to treat the excreta. On the other hand, if the amount of calcined lime used in this case exceeds 200 parts by mass, an excessive amount of heat is generated and cause the surface-treated calcined lime active instantly, thus causing creating lumpy substances to develop and unreacted substances to remain.

However, the amount of heat generating agent to be used should not be limited by the amount of calcined lime to be used, but rather be adjusted arbitrarily depending on the type and amount of the coating agent as well as the type of the heat generating agent to be used.

When calcined lime is used as the heat generating agent, its shape is not particularly specified, but it is preferably powdery. Its average particle diameter is preferably 0.1 µm - 5 mm, and more preferably 0.5 µm - 2 mm.

Moreover it is possible to use said surface-treated calcined lime as the heat generating agent, when the calcine lime surface-treated with said hydrophobic coating agent does not cause any aggregation due to its reaction with water, etc., during the excreta treatment, and also it is efficiently pulverized as a result of the mixing condition of the excreta treatment, or when the coating agent peels off from the surface of the calcined lime surface-treatedwith the coating agentdue to the physical and/or chemical mechanism described in the section of the hydrophobic coating agent, so that the reaction between the calcined lime with its activated surface being exposed and excreta provides a sufficient amount of heat necessary for melting the coating agent existing on the surface-treated calcined lime used in this invention.

### (Water-absorbent member)

The excreta treatment agent according to the present embodiment is preferable further to contain a water-absorbent member. The water-absorbent member to be used in the present invention is added from the standpoint of suppressing the reaction between active calcined lime and excreta, i.e., from the standpoint of suppressing water contents contained in the excreta react with the activated surface of the calcined lime all at once, thus generating lumpy substances due to aggregation and causing untreated excreta to remain. Although said water-absorbent member is not specifically limited and any known substance can be used so long as it does not adversely affect the excreta treatment agent and the excreta treatment method, it is preferably water-absorbent and allows the absorbed water to react with the calcined lime when its activated surface makes contact with the water. Examples of water-absorbent members include, for example, saw dust, water-solvent paper, fiber bundles, unwoven fabrics, peat moss, and peat. These materials can be used singularly or as a combination of two or more. The amount of the water-absorbent member to be used can be adjusted in accordance with the type and shape of the water-absorbent member and the water content of excreta which is the target of the treatment, but it is preferably 1-10 parts by mass, or more preferably 2-6 parts by mass relative to 100 parts by mass of the excreta treatment agent.

### (Additives)

For the excreta treatment agent to be used in the present invention, other additives can be used in place of or added to the water-absorbent member so long as it does not adversely affect the excreta treatment agent and the method of excreta treatment using it. Examples of such additives include perfumes, fresheners, and deodorants from the standpoint of countermeasures against odors; hydrophilic organic compounds such as alcohol and surfactants from the standpoint of improving water wetness of said surface-treated calcined lime; desiccants such as silica gel, anhydrous sodium sulfate or water retention agents such as water-absorbent plastics from the standpoint of controlling water content of the excreta. These additives can be used singularly or as a combination of two or more.

Examples of said perfumes include, for example, lemon oil, lemon grass, cinnamon oil, lavender oil, and vetiver oil.

Various surfactants can be used as the aforementioned surfactant, and specific examples include, for example, nonionic-group surfactants such as poly (oxyethylene) alkyl ether, poly(oxyethylene) alkyl phenyl ether, poly(oxyethylene) mono-fattyacidester, poly(oxyethylene) di-fatty acid ester, poly(oxyethylene) propylene glycol fatty acid ester, poly(ethylene) sorbitan mono-fatty acid ester, and glycerin mono-fatty acid ester; anion-group surfactants such as salt of alkyl benzene sulfonic acid, alkyl sulfosuccinate, alkyl sulfate, poly(oxyethylene) alkyl sulfate, and aryl sulfonate; cation-group surfactants such as salt of long-chain primary amine, salt of di-alkyldi-methylammonium, salt of alkyltrimethylammonium, benziltrimethylammonium chloride, salt of alkylpyridinium, benzalkonium chloride, and benzethonium chloride; and amphoteric surfactant such as alkyldiaminoethyl glycine hydrochloride, and alkylpolyamino ethyl glycine hydrochloride.

Also, wrappers made of water-soluble plastics, wrappers made of water-soluble paper, or wrappers made of water-decomposable unwoven fabrics, all of which are to be used in applying the excreta treatment agent to the excreta and which are to be described later, can be included as a part of the additives of the excreta treatment agent of the present invention.

### (Method of adjusting excreta treatment agent)

The method of adjusting the excreta treatment agent according to the present invention is not specifically limited and a method of mixing calcined lime surface-treated with a hydrophobic coating agent with a heating agent and mixing them with the water-absorbent member and/or other preferable additives if needed can be applied for it.

Since said surface-treated calcined lime is coated with the hydrophobic coating agent, said surface-treated calcined lime has not only an increased moisture resistance of the excreta treatment agent, but also a delayed reaction capability that prevents it from reacting instantly when it comes in contact with the excreta during the excreta treatment, so that it does not produce lumpy substances which might otherwise be caused as the excreta come in contact immediately with the activated surface of a calcined lime. Therefore, the excreta treatment agent according to the present invention can be evenly distributed over excreta. However, if higher fatty acid, particularly higher fatty acid with a carbon number of 15-26, is used as said coating agent, the hydrophobicity may become higher. In such a case, additives such as a surfactant or a salt of higher fatty acid with a relatively low hydrophobicity, in particular, a salt of a higher fatty acid with a carbon number of 8-14, can be added to the excreta treatment agent.

Although the method of sprinkling said excreta treatment agent 20 over excreta is not specifically limited, for example, a method of preparing said excreta treatment agent 20 in a wrapper and applying said wrapper over the excreta during the treatment is preferable from a standpoint such as ease in handling. The wrappers used in this case are preferably wrappers made of water-soluble plastics, wrappers made of water-soluble paper, or wrappers made of water-decomposable unwoven fabrics. Including all these aspects, said excreta treatment agent 20 should be preserved under a humidity-resistant and water-resistant condition.

In the present embodiment, said excreta treatment agent 20 is simply sprinkled over the excreta received in the toilet pack 10 and no positive mixing (agitation) with the excreta is applied. Since the excreta treatment agent 20 and the excreta are not positively agitated, the reaction temperature rises gradually. Although the excreta do not become powdery, they turn into a dried state after a few days producing no foul odor.

Fig. 6 is a schematic view showing a treatment bag according to the embodiment of the present invention. Fig. 7 is a schematic view showing a condition where the excreta is thrown into the treatment bag according to the embodiment of the present invention together with the toilet pack.

With reference to Fig. 6 and Fig. 7, the treatment bag 30 according to the present embodiment is a simplified throwawaybag for thermally treating excreta, which is formed into, for example, a 390 mm x 470 mm bag shape by vertically folding a thermally resistant film material 30A and jointing the left and right edges 30B. This treatment bag 30 is preserved in a folded condition, while its opening part 31 is expanded when it is used so that excreta and the excreta treatment agent 20 can be thrown into it together with the toilet bag 10.

In the present embodiment, the film material (base material) of the treatment bag 30 is made of the same material as that of the toilet pack 10, a metal foil, e.g., aluminum foil or copper foil. Since it is made of a metal foil same as the toilet pack 10, it provides heat resistance for a temperature range of 80°C to 450°C and hence withstands the reaction heat of the excreta treatment agent 20, thus eliminating concerns for making holes or causing fires due to the heat.

Although the material of the treatment bag 30 is water-repellent as it is made of a metal foil regardless of whether it has plastic layers on the front and back surfaces or not, in the present embodiment, the front and back surfaces of the metal foil material are coated or laminated with layers of plastics, e.g., polyethylene, polypropylene, nylon, etc., similar to the toilet pack 10. Therefore, it is possible to thermally fuse the bag using a sealer (sealing device) in forming the bag into a container shape, thus providing an excellent sealing capability of the jointed area. Additionally, although the plastic layer is laminated on both the front and back surfaces of the metal foil material in the present embodiment, the invention is not limited to such a configuration; it only requires a plastic layer to be laminated on the jointing surface of it.

The opening part 31 of the treatment bag 30 is formed to be sealable. For example, a fastener 32 is provided on the inside of the opening edge area of the treatment bag 30 consisting of a linear convex part and a linear concave part into which the convex part is inserted to be held thus allowing easy sealing and opening. The user can easily seal the opening part 31 of the treatment bag 30 by pressing down the fastener 32 with a finger and inserting the linear convex part into the linear concave part.

For example, a ventilation window part 33 of a 50 mm x 170 mm rectangular shape is provided in the vicinity of the opening area of the treatment bag 30, while activated charcoal 42 for adsorbing foul odor gas inside the treatment bag 30 and a water-absorbent member 41 forpreventingmoisture absorption of the activated charcoal are installed in the ventilation window part 33. The method of installing the activated charcoal 42 and the water-absorbent member 41 to the ventilation window part 33 can be done by gluing or thermally fusing them to the opening peripheral area of the ventilation window part 33.

The water-absorbent member 41 is a water-absorbing film-like material formed to match the opening shape of the ventilation window part 33, and is mounted on the ventilation window part 33. The material of the water-absorbent member 41 can be unwoven fabrics, porous acrylic plastics, polymer moisture-absorbent agent, etc. The material should preferably be a kind that does not transform with the heat of steam.

The activated charcoal 42 is, for example, an activated charcoal sheet, and is installed on the water-absorbent member 41 to cover the ventilation window part 33. The activated charcoal 42 is specifically a kind where activated charcoal fabric is woven into an unwoven fabric in a sheet-like shape using polyethylene (PE) or polypropylene (PP) as a binder. The water-absorbent member 41 and the activated charcoal 42 can be used as an integral unit or separate units. The placements of the ventilation window part 33, the water-absorbent member 41, and the activated charcoal 42 are not specifically limited in the present embodiment.

### (Excreta treatment method)

Next, let us describe a preferred embodiment of the method of treating excreta according to the present invention with reference to Fig. 1 through Fig. 7.

In the method of treating excreta according to the present embodiment, excreta is received by the toilet pack 10 formed in a container shape as described above. The toilet pack 10 is normally stored in a folded condition and is taken out from a storage area or an emergency bag etc at a time of a disaster. As shown in Fig. 3 and Fig. 4, the toilet pack 10 is formed in a container shape by holding the rectangular holding parts 13 and 14 located on both sides of the outside of the toilet pack 10 with fingers and expanding its opening edge 11 sideway, and the toilet pack 10 is held by the toilet seat 51 of an existing clamshell type toilet (Western style toilet) 50 with the help of the holding parts 13 and 14.More specifically, as shown in Fig. 3, the container portion 15 of the toilet pack 10 is placed inside the toilet 50 while the toilet seat 51 of the toilet 50 is lifted open, and the holding parts 13 and 14 which are expanded by pulling left and right with fingers are placed on the left and right edges of the toilet 50. Next, as shown in Fig. 4, the toilet seat 51 is brought down to clamp the holding parts 13 and 14 between the left and right edges of the toilet 50 and the toilet seat 51, thus securely holding them.

Incidentally, the toilet pack 10 can be used by itself without installing it into the existing toilet bowl, or can be used in a toilet without the toilet seat (Japanese style toilet), although it cannot be held.

The toilet pack 10 is formed by thermally fusing the edges of a metal foil laminated on the front and back sides with plastic layers to make it water-repelling as a whole including the jointed areas, so that there is no chance for the water contents of the excreta received by the toilet pack 10 to leak outside of the toilet pack 10.

The treatment bag 30 shown in Fig. 6 should preferably be placed outdoors, e.g., verandas, spaces under the eaves, or bath rooms which are shelter from the rain. After receiving excreta, the toilet pack 10 is moved to the place where the treatment bag 30 is locate, the bag 21 in which the excreta treatment agent 20 is stored is opened as shown in Fig. 5 to sprinkle the excreta treatment agent 20 over the excreta in the toilet pack 10. The excreta being treated can be only solid waste or a mixture of solid waste and urine.

Although the amount of the excreta treatment agent 20 to be used can be measured each time when it is used, it is preferable to portion the amount to be used for one time or several times of defecation and pack it in the bag 21 considering the convenience of usage. More specifically, the portion should preferably be 1-200 parts by mass, or more preferably be 10-150 parts by mass, or most preferably be 80-120 parts by mass relative to 100 parts by mass of the excreta. If the aforementioned amount of usage is less than 1 part by mass, the adsorption treatment of the excreta would be less than satisfactory and leave unreacted substances. On the other hand, if it is more than 200 parts by mass, the treatment efficiency would be less than satisfactory.

After sprinkling the excreta treatment agent 20, the excreta and the excreta treatment agent 20 are thrown into the treatment bag 30 without mixing (agitating) them together with the toilet pack 10 to treat the excreta thermally inside the treatment bag 30.

After throwing the excreta and the excreta treatment agent 20 together with the toilet pack 10 into the treatment bag 30, the opening part 31 is closed using the fastener 32, and they are left to cause the hydration reaction ( exoergic reaction) between the excreta and the excreta treatment agent 20 inside the treatment bag 30. As the reaction between the heat generating agent and the excreta generates heat, the coating agent of the surface-treated calcined lime melts and the activated surface of the calcined lime is exposed, so that the calcined lime reacts with water contained in the excreta to generate heat and the calcium hydrate which is the reaction product adsorbs the excreta. In the present embodiment, said excreta treatment agent 20 is simply sprinkled over the excreta received in the toilet pack 10 and no positive mixing (agitation) with the excreta is applied, so that the reaction temperature rises gradually. More specifically, the temperature rises to approximately 80°C within a few minutes and then drops down to the normal temperature within 10 minutes or so. Although the excreta do not become powdery, they turn into a dried state after a few days producing no foul odor. Since there is no agitation of the excreta treatment agent 20 and the excreta, there is no need for an agitation tool such as a paddle or labor. The dried excreta after the treatment is hydrophobic because of the effect of the coating agent, so that it does not return to the original state of the excreta due to mixing of water such as rain water. Moreover, since the temperature inside the treatment system reaches approximately 80°C and the inside of the treatment system has a strong alkalinity, bacteria in the excreta are alkaline-sterilized, and it is expected that no foul odor will develop from treated substances even after five or six months.

Although the treatment bag 30 will be filled with vapor due to the exoergic reaction of the excreta treatment agent 20, it is only necessary to open the opening part of the treatment bag 30 to disperse the vapor if an excessive amount of the vapor is generated and it is only necessary to close the opening part 31 of the treatment bag 30 after the vapor generation subsides.

Since the toilet pack 10 and the treatment bag 30 are made of metal foils and they are heat resistant to the temperatures above 80°C and below 450°C, holes or fires due to the heat generated by the reaction of the excreta treatment agent 20 are prevented. Moreover, the activated charcoal 42 for adsorbing the foul odor gas generated in the treatment bag 30 is installed to the vicinity of the opening part of the treatment bag 30. The activated charcoal 42 is also combined with the water-absorbent member 41, which prevents the drop of the foul odor gas adsorption activity due to clogging of the porosity of the activated charcoal 42 prematurely by steam vapor.

The treated objects (treated excreta etc) are discarded when the thermal treatment inside the treatment bag 30 is completed. Practically speaking, the reaction heat of the thermal treatment subsides to a temperature close to the normal temperature in about 10 minutes after throwing the contents of the toilet pack 10 together with it into the treatment bag 30 making it possible to discard them. The treated objects (the treated excreta etc) can be discarded hygienically as the thermally treated excreta and the toilet pack 10 etc are contained in the treatment bag 30

As can be seen from the above, according to the excreta treatment device 1 and the method of treating excreta according to the present embodiment, it is possible to receive excreta by the throwaway toilet pack 10 placed on the existing regular toilet 50, and conduct a thermal treatment of the excreta in the treatment bag 30 placed outdoors, for example, using a excreta treatment agent containing a heat generating agent and calcined lime surface-treated with, a hydrophobic coating agent. The container-shaped toilet pack 10 for receiving excreta is formed by thermally fusing a metal foil laminated on the front and back sides with plastic layers to make it water-repelling as a whole, so that there is no chance for the water contents of the excreta received by the toilet pack to leak outside of the toilet pack 10. Moreover, since the part of the toilet pack which receives excreta has a heat insulating material 12 built into it, the excreta treatment agent 20 is sprinkled over the excreta received by the toilet pack 10, and the toilet pack 10 containing all those things is thrown into the treatment bag 30 made of a metal foil, so that a hole or a fire due to the heat generated by the reaction of the excreta treatment agent 20 is prevented. Further, the thermally treated excreta can be discarded together with the treatment bag 30 so that it is extremely hygienic.

The present invention is not limited to any of the embodiments described above, but rather can be modified within the range of the claims of the patent. For example, the excreta treatment agent is not specifically limited to the embodiments described above but rather any kind of excreta treatment agent such as a excreta treatment based on calcined lime.

The folding-formed structure of the toilet pack 10 is not limited to the embodiment described in the above, but rather can be modified to have a bottom part with a flat surface provided in the container portion 15 to widen the container portion 15.

### [Industrial application]

The present invention can be preferably in toilets and in the field related to treatments of excreta.

### [Reference code]

- 1: Excreta treatment device
- 10: Toilet pack
- 10A: Film material
- 12: Heat insulation material
- 13, 14: Holding parts
- 20: Excreta treatment agent
- 30: Treatment bag
- 33: Ventilation window part
- 41: Water-absorbent member
- 42: Activated charcoal
- 50: Existing toilet
- 51: Toilet seat

## Claims

1. A method of treating excreta **characterized in** receiving excreta with a container-shaped toilet pack made of a heat-resistant and water-repellent film material containing a heat insulating member at least in the excreta receiving area;
sprinkling excreta treatment agent over the excreta received by said toilet pack;
throwing them without mixing into a treatment bag made of a heat-resistant film material together with said toilet pack to have the excreta thermally treated inside said treatment bag; and
discarding the contents together with the treatment bag.

2. The method of treating excreta claimed in Claim 1 where said toilet pack is formed by thermally fusing a metal foil laminated with a plastic layer into a container shape, and the heat insulating member is embedded at least in its excreta receiving area.

3. The method of treating excreta claimed either in Claim 1 or Claim 2 where said toilet pack is placed in an existing toilet bowl to receive excreta.

4. The method of treating excreta claimed in either one of Claim 1 through Claim 3, wherein said treatment bag is formed by thermally fusing a metal foil material laminated with a plastic layer into a bag shape, with its opening part formed in such a way as to make it sealable.

5. The method of treating excreta claimed in Claim 4 wherein said treatment bag has a ventilation window part, which ventilation window part is equipped with activated charcoal for absorbing foul odor gas existing in the treatment bag and a water-absorbing material that prevents said activated charcoal from absorbing moisture.

6. The method of treating excreta claimed in either one of Claim 1 through Claim 5, wherein said excreta treatment agent is at least a powdery agent containing a heat generating agent and calcined lime surface-treated with a hydrophobic coating agent.

7. A device for treating excreta **characterized in** comprising a container-shaped toilet pack made of a heat-resistant and water-repellent filmmaterial containing a heat insulating member at least in the excreta receiving area;
excreta treatment agent to be sprinkled over the excreta received by said toilet pack; and
a treatment bag made of a heat-resistant film material, into which said toilet pack is thrown after the sprinkling of said excreta treatment agent to thermally treat the excreta.

8. The device for treating excreta claimed in Claim 7 where said toilet pack is formed by thermally fusing a metal foil laminated with a plastic layer into a container shape, and the heat insulating member is embedded at least in its excreta receiving area.

9. The device for treating excreta claimed in Claim 7 or Claim 8 wherein said toilet pack has holding parts that can be held by the toilet seat of an existing clam-shell type toilet.

10. The device for treating excreta claimed in either one of Claim 7 through Claim 9, wherein said treatment bag is formed by thermally fusing a metal foil material laminated with a plastic layer into a bag shape, with its opening part formed in such a way as to make it sealable.

11. The device for treating excreta claimed in Claim 10 wherein said treatment bag has a ventilation window part, which ventilation window part is equipped with activated charcoal for absorbing foul odor gas existing in the treatment bag and a water-absorbing material that prevents said activated charcoal from absorbing moisture.

12. The device for treating excreta claimed in either one of Claim 7 through Claim 11, wherein said excreta treatment agent is at least a powdery agent containing a heat generating agent and calcined lime surface-treated with a hydrophobic coating agent.
